# NEW EUROPEAN PATENT SPECIFICATION

(11) **EP 0 608 235 B2**
(45) Date of publication and mention of the opposition decision: **04.05.2005**
(45) Mention of the grant of the patent: 10.01.1996
(21) Application number: 92913443.5
(22) Date of filing: 13.05.1992
(51) Int. Cl.: C12Q 1/56, G01N 33/86

(54) **METHOD FOR THE DIAGNOSIS OF BLOOD COAGULATION DISORDERS**
VEFAHREN ZUR DIAGNOSE VON STÖRUNGEN DER BLUTGERINNUNG
METHODE POUR DIAGNOSTIQUER DES ANOMALIES DE LA COAGULATION DU SANG

(30) Priority: 13.11.1991 SE 9103332; 20.12.1991 US 811303
(43) Date of publication of application: 03.08.1994
(73) Proprietor: T.A.C. Thrombosis and Coagulation Aktiebolag, 217 74 Malmö (SE)
(72) Inventor: T.A.C. Thrombosis and Coagulation Aktiebolag, 217 74 Malmö (SE)
(74) Representative: TER MEER STEINMEISTER & PARTNER GbR
(86) International application number: PCT/SE1992/000310
(87) International publication number: WO 1993/010261

(56) References cited:
- EP-A- 0 434 377
- EP-A1- 0 711 838
- WO-A-91/01383
- WO-A-91/02812
- WO-A1-91/01382
- WO-A1-96/15457
- SE-B- 464 135
- The New England Journal of Medecine, Vol.330, p.566-567, (1994)
- The Lancet, Vol.342, p.1501-1502, (1993)
- Nature, Vol.369, p.14-15, (1994)
- New England Journal of Medecine, Vol.330, p.517-522, (1994)
- Thrombosis and Haemostasis, Vol.70, p.1067-1071 (1993)
- Amer et al., Thrombosis Research (1990), 247-258
- Mitchell et al., New Engl.J.of Medicine (1987), 1638-1642
- Faioni, Thrombosis and Haemostasis (1991), 420 - 425
- Hoogendoorn, Blood (01.11.1991), 2283-2290
- Bertina, Res.Clin.Lab. (1990), 127-138
- Blombäck et al., Haemostasis and Thrombosis (1987), 967-981
- Bertina, Nature (1994), 64-67
- Dahlbäck, Advances in Genetics,"Thrombophilia" (1996), 135-175
- Svensson et al., New Engl. J. Medicine (1994), 517-522
- I.D. Walker "Guidelines on The Investigation and Management of Thrombophilia", J.Clin. Pathol. (1990), 703-710
- THROMB. HAEMOST., 65, Abstract 39, 1991, BJORN DAHLBACK et al., "Factor VIII Defect Associated with Familial Thrombophilia", page 658.
- Dahlbäck et al., Thrombosis and Haemostasis 65, 658 (1991), Abstract 39

## Description

The present invention concerns a novel method appropriate for diagnosis of thromboembolic diseases, e.g. hereditary thrombophilia. The invention can also be used for determining the risk for thrombosis in 'pregnant individuals, individuals undergoing surgery, individuals taking anti-conception drugs etc.

The blood coagulation comprises a complex system of inter-linked proenzymes, enzymes and cofactors performing its role at the surfaces of activated platelets and endothelial cells. When the system is activated the ultimate result is the formation of a blood clot containing insoluble fibrin. The initiation and termination of fibrin formation is carefully regulated in normal homeostasis. In vitro, platelets and endothelial cell surfaces are usually replaced with suitable phospholipids. For the invention the relevant part of the coagulation system is:

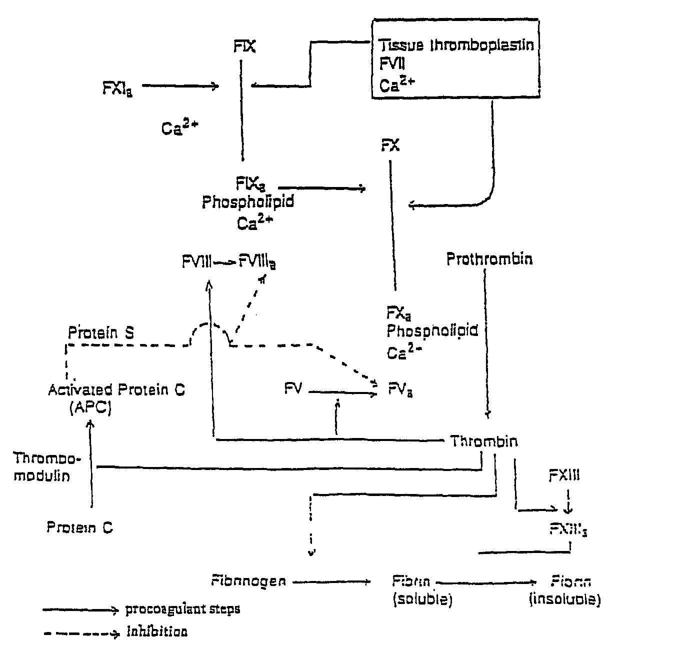

The important part for the invention relates to Protein C(= PC) and effects produced by action of activated Protein C (= APC) on the coagulation system. Protein C is a zymogen that in vitro may be activated by thrombin (= Factor IIₐ) alone, the combination thrombin-thrombomodulin, certain snake venoms, such as from Akistrodon Contortrix Contortrix, or purified Factor Xₐ. Activation of sample endogenous Protein C or addition of exogenously activated Protein C to a plasma sample will neutralise Factors Vₐ and VIIIₐ (degradation) and lead to prolonged times for blood coagulation in plasma samples of healthy individuals. Factors V and VIII are activated by small amounts of thrombin or Factor Xₐ. Protein S is a cofactor to Protein C.

Hereditary heterozygous deficiences in Protein C, Protein S and Antithrombin III (ATIII, a coagulation factor opposing coagulation) are found in approximately 10-15% of patients with diagnosed thromboembolic disease before the age of 40. Homozygous Protein C and S deficiencies are life threatening and affected individuals develop generalized microvascular thrombosis and purpura fulminance in the neonatal period.

Deficiencies in Protein C and S and ATIII are measured by both functional and immunological methods. One way of determining functional Protein C activity involves the steps: mixing the plasma sample to be tested with an excess of human plasma deficient in Protein C and addition of a Protein C activator and monitoring the appropriate substrate conversion. Highly specific assays have been achieved by using Protein C specific substrates. Alternatively one has also utilized substrates for enzymes, e.g. thrombin and Factor Xₐ, which activities are influenced by activated Protein C activity (i.e. enzyme activities that are generated or modulated by APC). In certain cases Protein C assays have involved isolation of the zymogen and a subsequent activation and addition of substrate to the activated form. Measurement of Protein C activity in plasma samples has also been suggested to be performed directly in the sample without addition of plasma deficient in Protein C. However, such methods will not discriminate an abnormality related to Protein C as such from a disorder related to factors interfering with the effects caused by Protein C (WO-A-91/02812).

Addition of activated Protein C to a plasma sample of a patient and study of the effect produced has been claimed to discover a defect Factor VIIIₐ molecule that is not degraded by activated Protein C (B. Dahlbäck and M Carisson, Thromb. Haemost. 65, Abstract 39, 658 (1991)). However, the data given in the present specification surprisingly indicate that the patient in question could not carry a defect Factor VIII/VIIIₐ.

In order to determine Protein S functional activity in a plasma sample the most common methods involve mixing the plasma sample with activated Protein C, an excess of Protein S deficient plasma and further reagents necessary to achieve clotting (Waart et al., Thromb. Res. 48, 427-37 (1987); Suzuki et al., Thromb. Res. 49, 241-51 (1988); Bertina et al., Thromb. Haemost. 53, 268-72 (1985); and Comp et al., J. Clin. Invest. 74, 2084-88 (1984). It has also been suggested to measure Protein S by incubating the plasma sample with Factor IXₐ and activated Protein C and measurement of the clot time or the conversion of a chromogenic thrombin substrate (KabiVitrum AB (S. Rosen) WO-A-9101382).

The present inventor has realized that there is hitherto an unrecognized thromboembolic disorder that can be diagnosed by the addition of activated Protein C to a patient sample containing coagulation Factors and measurement of an enzyme activity that is influenced by the APC added. The experimental results now presented indicate that the disorders in question are related to a hitherto unknown coagulation Factor(s) or unknown interactions of known Factors. The unknown Factor is not Factor Vₐ or VIIIₐ that are resistant to degradation by APC, or an inhibitor of the immunoglobulin-type for APC. The disorders are neither related to Protein S deficiency. For simplicity reasons the unknown Factor (s)/interaction(s) will be referred to as an unknown Factor in this text.

The samples assayed are plasma samples. The invention will be illustrated in relation to plasma samples.

Accordingly the present invention is concerned with an in vitro method for diagnosing in a human thromboembolic diseases caused by, or for determining the risk for a human to acquire manifestation of, a blood coagulation disorder designated APC resistance and recognized by an abnormally low anticoagulant response to exogenous activated Protein C (abbreviated APC) even in presence of normal levels of functional Protein S, Factors Va and VIIIa which are normally degraded by APC, and absence of lupus anticoagulants, said method comprising determining for a plasma sample comprising coagulation factors and derived from a human, the anticoagulant activity of exogenous APC by measuring the substrate conversion rate obtained for a blood coagulation enzyme, the activity of which is influenced by APC, by the following steps: (I) incubating said plasma sample with
(1) exogenous APC, or exogenous Protein C together with current exogenous reagents to transform the exogenous Protein C to APC; wherein the concentrations used in the final assay medium being 25 ng/mL-10 µg/mL for human APC, 10 ng/mL-50 µg/mL for non-human APC, and 5 ng/mL-5 µg/mL for bovine APC used in combination with 100 ng/mL-20 µg/mL bovine protein S;
(2) an exogenous Reagent (I), which at least partially activates the blood coagulation system of said sample and is selected in a manner known per se to cause activation of a coagulation factor used for the measurement in step (ii) ;
(3) components, such as phospholipid(s) and Ca⁺⁺ salt that are necessary for efficient reaction of the activated coagulation factors introduced by step (I) (2); and, if desired,
(4) an exogenous substrate for an enzyme, the activity of said enzyme being influenced by APC;
   (ii) directly measuring said substrate conversion rate obtained in(i), and
   (iii) comparing the conversion rate measured in step (ii) with a standard value obtained from samples from normal individuals, which samples have been subjected to steps (i) and (ii) under the same conditions as the plasma sample from said human;
in which method a substrate conversion rate obtained for a plasma sample in step (ii), that is higher than the standard value indicates that said human suffers from or runs the risk of acquiring manifestation of said disorder.

In case the substrate conversion rate is not normal compared to the standard, the individual from which the sample derives is classified as suffering from the disorder or being at risk for acquiring manifestation of the disorder. An increased conversion rate of the sample indicates a thromboembolic disease or a riskfor such a disease (with fibrinogen as the substrate an increased conversion rate means a shortened clotting time). The significance of a lowered conversion rate is at the present stage not known (with fibrinogen as the substrate a lowered conversion rate means a prolonged clotting time). Probably it is not related to any disease.

The range of the normal conversion rate may be quite broad. Hence, it might, as a complement, be of value to run steps (i)-(ii) on a plasma sample from the individual with exclusion of the incubation according to (i)(1) and compare the result obtained with that obtained according to the invention.

The incubation according to (i)(2) serves to introduce an activated coagulation factor that can be used for the measurement in step (ii). The expression "partially" means that the addition of Reagent (I) leads to the presence of at least Factor IXₐ. Reagent (I) may be a certain coagulation factor or a reagent that activates the system via the intrinsic or extrinsic pathway. Accordingly Reagent (I) may be Factor IXₐ or Factor XIₐ (intrinsic pathway), Factor XIIₐ (intrinsic pathway), kallikrein (intrinsic pathway), a contact activator (intrinsic pathway) such as kaolin, celite or ellagic acid (intrinsic pathway), an APTT reagent (Activated Partial Thromboplastine Time; i.e. a reagent containing a phospholipid and a contact activator (intrinsic pathway)), tissue thromboplastin (PT-reagent, PT = Prothrombin time, (extrinsic pathway)). In cases where a poor specificity is acceptable Reagent (I) may also be Factor Xₐ.

Protein C (i)(1) may be of various origin. In case the Protein C and the sample are of different species origin it is highly recommendable to include Protein S (cofactor to activated Protein C) in the incubation mixture. Protein C and Protein S should be of the same species origin, for instance bovine Protein C requires bovine Protein S. Protein C is preferably activated prior to being added, although activation may also be accomplished after it has been added to the sample. Activation shall take place under standardized and defined conditions. Normal activation agents are those given on page 2. Recambinantly produced biologically functional forms of Proteins C and S can also be used.

The components used according to step (i)(3) depend on the mode employed and may necessitate the inclusion of plasma protease inhibitors for enzymes other than the monitored one or of a fibrin polymerization inhibitor. Ca²⁺ may be in the form of a plasma soluble salt that provides the Ca²⁺ ion in free uncomplexed form, i.e. strong Ca²⁺ chelators should be avoided. In the final assay medium the concentration of Ca²⁺ may be selected within 0.5-50 mM, preferably within 5-15 mM, such as 6-7 mM. Too high a concentration may inhibit the coagulation system.

The substrate according to (i)(4) is normally a synthetic substrate for an enzyme which activity is influenced by activated Protein C, e.g. thrombin (= Factor IIₐ) and Factor Xₐ. Suitable synthetic substrates are water soluble and have preferably oligopeptide structure with three, four or five amino acid residues and an amino terminal that is protected from being attacked by amino peptidases. The protection is accomplished either by a protecting group or by having a D-amino acid in the amino terminal. In order to give a detectable response the carboxy terminal of a synthetic substrate is amidated with a group that specifically can be released and detected upon action of the relevant blood coagulation protease. The group to be released is selected among chromogenic, fluorogenic or chemiluminogenic groups and other analytically detectable groups. Se further H.C. Hemker, "Handbook of synthetic substrates for the coagulation and fibrinolytic system", Martinus Nijhoff Publishers, 1983, and J. Fareed et al, "Synthetic peptide substrates in hemostatic testing" in CRC Critical Reviews in Clinical Laboratory Sciencies Vol 19, issue 2, 71-134(1983).

The order of addition and the incubation vary with the mode of the invention. For instance in case Reagent (I) is an APTT reagent (i)(2) and the substrate conversion to be monitored is fibrinogen to fibrin, reagent (I) is added to the sample and allowed to maximally activate Factor XI to Factor XIₐ. Then Ca²⁺ (i)(3) is added and the time for clotting measured. Activated Protein C according to step (I:b) is introduced either simultaneously with, prior to or after the activation to Factor XIₐ. A PT-assay is performed similarly with addition of tissue thromboplastin (instead of the APTT reagent) to the sample in an amount sufficient for activation of Factor X to Factor Xₐ or Factor IX to Factor IXₐ. Thereafter activated Protein C (i)(1) is added and finally the clotting time is measured as in any APTT assay. In case a synthetic substrate is used it can be added at any stage before or at the start of the monitoring reaction. In order to run the monitoring reaction with high specificity, the above-mentioned inhibitors may be introduced at any suitable stage into the reaction medium. For instance it may be appropriate to add a thrombin inhibitor together with a substrate for Factor Xₐ, when Factor Xₐ activity is measured. The same inhibitor added prior to addition of the substrate may, however, adversely affect the formation of Factor Xₐ.

In order to accomplish a specific result with respect to the above-mentioned unknown Factor one should try to keep the patient plasma sample content of the final assay medium as high as possible. Accordingly patient plasma sample content in tests having good specificity should be >10 %, in particular >20% or >35% (v/v).

It may be practical to sell and use reagents according to (i)(1-4) in predispensed combinations that may have been lyophilized separately or as mixtures containing at least two of the components given in (i)(1-4), preferably in the doses used for testing. It may also be practical to have performed the lyophilization in the vial to be used in the assay. Suitable combinations are (concentration ranges refer to values during the assay, preferred ranges are given within brackets):

### A. APTT based clot methods and APTT depending clot methods for factors V and VIII.

| | |
|---|---|
| 1. Human APC | |
| | 25ng/mL-10µg/mL |
| 2 APC species (non-human) | |
| | 10ng/mL-50µg/mL |
| 3. Bovine APC/Bovine Protein S, from other non-human species. | APC: 5ng/mL-5µg/mL |
| | Protein S: |
| | 100ng/mL-20µg/mL |
| | (10ng/mL-20µg/mL) |

All reagents given in 1-3 above and intended to be used in the invention may be lyophilized in the absence or presence of Ca²⁺. If present, the amount of Ca²⁺ should give a Ca²⁺ concentration of 0.5-30mmol/L in the final assay medium. Phospholipid may be included in the lyophilized preparations.

### B. APTT modified clot methods in which contact factor activation has been excluded.

| | |
|---|---|
| Factor IXₐ | 0.05 ng/mL-2µg/mL |
| Factor XIₐ | 0.05 ng/mL-2µg/mL |
| Factor XIIₐ | 0.05 ng/mL-2µg/mL |
| Kallikrein | 0.05 ng/mL-2µg/mL |

No limitation regarding species. FIXₐ may also be used together with any of the combinations A 1-3 including the presence or absence of Ca²⁺ and phospholipid.

### C. APTT chromogenic methods.

Combinations according to A 1-3 with inclusion of fibrin polymerization inhibitor (concentration >/= Kₗ, Kₗ = inhibition constant) and chromogenic substrate (concentration >/= 0.1Kₘ, Kₘ = Michaelis-Mentens constant). Alternatively the chromogenic substrate is lyophilized separately or in the presence of Ca²⁺ that in turn optionally is combined with a fibrin polymerization inhibitor, As another- alternative, the substrate is lyophilized together with a fibrin polymerization inhibitor but in the absence of Ca²⁺. The constituents shall provide conditions such that no disturbing substrate hydrolysis takes place during reconstitution.

### D. APTT modified chromogenic methods in which contact factor activation has been excluded.

Combinations of reagents as given under B and C.

### E. PT clot method utilizing tissue thromboplastin.

Reagents according to A 1-3 optionally combined with ca²⁺ and/or tissue thromboplastin.

### F. Modified clot method for Factor v defect.

### Factor Xₐ 0.02ng/mL-0.5µg/mL

Factor Xₐ is not limited to species. The reagent may contain combinations according to A 1-3 optionally together with Ca²⁺ and/or phospholipid.

### G. PT chromogenic method.

Combinations according to C and E above but with thromboplastin instead of phospholipid.

### H. Chromogenic Factor VIII method.

Reagents according to A 1-3 employed in a standard Chromogenic Factor VIII assay. The reagents may have been lyophilized together with either of Factor IXₐ +/- Ca²⁺ +/- phospholipid or Factor X +/- Ca²⁺ +/- Phospholipid, with Factor X concentration of 0.1µg/mL-50µg/mL. The reagent may also comprise inclusion of small amounts of thrombin and, when Factor X is included, also Factor IXₐ. Furthermore, a chromogenic substrate for Factor Xₐ may be included in the reagent.

### I. Chromogenic Factor V method.

Reagents according to C and F with or without inclusion of prothrombin (0.02ng/mL-50µg/mL) APTT reagents may be included in combinations A-D, provided they are not co-lyophilized with Ca²⁺. Active enzymes and their substrates may be co-lyophilized as recently described (EP-A-318,571).

The invention is primarily intended as a method in order to find individuals that need further diagnostication, but comprises also specific factor assays according to F, H and I above. The proper selection of reagent (I) and substrate to be monitored (i)(4) refine the possibility of finding where in the coagulation system a diagnosed disorder is located. In principle the inventive method will detect disorders related-to defective interactions between activated Protein C and Factor Vₐ, Factor VIIIₐ. It will also detect the presence of inhibitors of activated Protein C, and abnormalities in hitherto unrecognized interactions and factors influenced by Protein C activation or activated Protein C activity.

The invention will now be illustrated by way of the inventor's discovery of a patient suffering from a novel disorder in the blood coagulation system.

### EXPERIMENTAL PART

### Case Report:

The proband is a male born in 1942. In 1961, he had the first episode of deep venous thrombosis in one of the legs. After this, he was healthy and free of thrombosis for almost 20 years. Between 1980 and 1987 he had multiple thrombotic episodes, occurring at least once a year. The thrombotic events were treated with vitamin K antagonists for up to three months. A thrombus was positively verified with flebography at least at two occasions. The proband has developed a post-thrombotic syndrome in his legs. He has no other disorders. In 1987, he quit smoking and at the same time he started taking aspirin daily. During 1987-1991 he has not experienced any thrombo-embolic episode. Both male and female members of the patient's family have similar histories with multiple episodes of deep venous thrombosis. His 10 year older brother have had deep venous thrombosis at multiple occasions, most of them occurring between the age of 45 and 50. The proband also reports that an uncle on his mother's side has had a medical record with multiple episodes of thrombosis. The patient's mother was born in 1905, and she has had episodes when deep venous thrombosis has been suspected clinically. Two more brothers and a sister have had incidences where thrombosis has been suspected.

Known coagulation and immunological methods used as a complement to the inventive method for the diagnosis.

The activated partial thromboplastin time (APTT, Organon Technica), Owren's P&P, thrombin time and reptilase time were determined with standard methods.Antithrombin III was measured with an amidolytic assay (Coatest® ATIII, Kabi Diagnostica, Mölndal, Sweden). Total and free Protein S and Protein C antigen levels were determined with previously described immunochemical methods (Malm J. et al., Br. J. Haemat. 68, 437-443 (1988). Protein C function activity was analysed with a synthetic substrate after activation with the venom from Agkistrodon Contortrix Contortrix using a commercially available kit (Coatest® Protein C, Kabi Diagnostica AB, Mölndal, Sweden).

Absorption of IgA, IgG and IgM was performed as previously described (Dahlbäck B. et al., Blood 62, 218-225 (1983).

A second functional Protein C assay was also performed as previously described (Hickton C.M., Thromb. Res. 41 501-8, (1986)). The method included barium-citrate absorption of plasma. The proteins that bound to the barium-citrate were eluted and the eluate was incubated with a thrombin-thrombomodulin complex to activate Protein C. The amount of APC after activation was quantified using an APTT clotting assay.

### Inventive methods

1. An APTT based method was used to determine the anticoagulant effect of purified APC in patient plasma. In this method (APC-APTT assay) the APC mediated prolongation of the APT-time was measured as follows: 0.1 ml plasma was incubated with 0.1 ml APTT reagent for 5 minutes at 37°C before addition of 0.1 ml of an APC-Ca²⁺ mixture (0-20 µg/ml APC in 10 mM Tris-HCl, 0.15 M Nacl, 30 mM calcium chloride pH 7.5 containing 0.1 % bovine serum albumin (BSA)) which initiated blood coagulation. The APC was prepared as previously described (Dahlbäck B. et al., J. Biol. Chem. 261, 12022-12027 (1986). The assay was run with bovine APC with or without the presence of bovine Protein S.
   Normally APTT assays are run without addition of Nacl. Accordingly, but also because Nacl prolongs the coagulation times, it is preferred to run this mode of the invention without addition of Nacl.
2. In order to determine the effect of APC on plasma Factor V, increasing concentrations of APC (10 µl diluted in 10 mM Tris-HCl, 0.15 M Nacl, pH 7.5 containing 0.1 % BSA) were added to 0.09 ml plasma. Immediately after the APC-addition, 0.1 ml rabbit brain cephalin (diluted in 0.15 M Nacl) and 0.1 ml 30 mM CaCl₂ were added. After incubation for 15 seconds at 37°C, clotting was initiated with 0.1 ml. Factor Xₐ (150 ng/ml diluted in 10 mM Tris-HCl, 0.15 M NaCl, pH 7.5 containing 0.1 % BSA). In the absence of APC, this Factor Xₐ concentration gave an approximate clotting time of 30 seconds in the control plasma used, Factor Xₐ was prepared as described previously (Dahlbäck B. et al., J. Biol. Chem. 261, 12022-12027 (1986)).
3. A modification of a commercial Factor VIII assay (Coatest® Factor VIII, Kabi Diagnostica AB, Mölndal, Sweden) was used to analyse the effect of APC on plasma Factor VIII. Patient or control plasma (25 µl 1/125 to 1/400 in 10 mM Tris-HCl, 0.15 M NaCl, pH 7.5 containing 0.1 % BSA) was incubated with 75 µl of the kit reagent containing Factor IXₐ, Factor X, phospholipid and Ca²⁺. Just prior to the test, increasing concentrations of APC (0.1-100 µg/ml) were added to this reagent. After 20 minutes incubation at 37°C, 50 µl of a mixture of the synthetic substrate (2.7 nM) (Bz-Ile-Glu(gamma-OR)-Gly-Arg-pNA=S-2222, Kabi Diagnostica, Mölndal, Sweden) and the thrombin inhibitor (60 µM) (N-dansyl-(p-guanidino)-Phe-piperidide=I-2581, Kabi Diagnostica AB, Mölndal, Sweden) was added. After incubation for 10 minutes more at 37°C, the reaction was interrupted by the addition of 150 µl (1 M) citric acid and the absorbance was measured at 405 nm. A standard curve for Factor VIII was made using normal plasma diluted from 1/100 to 1/800.

### Results of the methods used in the cause of the investigation.

The APT-time together with values for ATIII and Protein S were normal and the patient had no indications of the presence of lupus anticoagulants. The plasma level of Protein C was normal, both when being measured with the immunological method and with the functional assay, which included Protein C activation with snake venom and quantitation of APC with a synthetic substrate.

The barium-citrate functional Protein C assay gave consistently lower Protein C values for the eluate when diluted 1:10 compared to 1:40. This might indicate the presence of an inhibitor for APC. In order to check this, we used the inventive APC-APTT assay. The clotting time obtained was always shorter than for the control plasma. In order to rule out an inhibitor of the immunoglobulin type, the patient's plasma was depleted completely in IgA, IgG or IgM by absorption. The shortened clotting time did not disappear. The results found could be due to a functional Protein S deficiency. However, this possibility was ruled out since bovine APC, when added with or without bovine Protein S is considerably less efficient in prolonging the APT-time of the proband's plasma compared to prolongation in the control plasma.

A third possible mechanism for the observed APC-resistance was that the proband's Factor Vₐ or Factor VIIIₐ could be resistant to cleavage by APC. To elucidate this possibility, assays were devised which directly measured the inhibition of plasma factors Vₐ and VIIIₐ by APC. Using the Factor Xₐ based clotting assay (described above), the inhibition of patient Factor Vₐ by APC was found to be normal suggesting that Factor Vₐ in the patient's plasma was degraded in a normal fashion by exogenously added APC. This experiment ruled out the possibility of a Protein C inhibitory antibody explaining the APC-resistance. To test the remaining possibility, i.e. that APC could not degrade the proband's Factor VIIIₐ, the effect of added APC in a Factor VIIIₐ assay was tested. However, the proband's Factor VIIIₐ was found to be normally degraded by the added APC when compared to control plasma. This finding is contrary to the inventor's earlier publication (B. Dahlbäck and M Carisson, Thromb. Haemost. 65, Abstract 39, 658 (1991)).

To investigate whether the APC-effect was inherited, 18 family members were analyzed using the APC-APTT assay. 10 (both male and female) of the 18 tested family members did not respond to APC with normal prolongation of their clotting times, which suggests that the factor molecule responsible for the effect is resistant to APC. This result shows that the defect molecule was inherited and present in the family members that did not give normal prolongation of their clotting times. It is noteworthy that in the absence of added APC, the APT-times of these individuals and of the proband were shorter than for the controls. This may suggest partial degradation of relevant Factor molecules during APTT assays of normal plasma. To test the sensitivity of the APC-APTT assay for the presence of APC-resistance, mixtures of proband and normal plasma (1:1, 1:10 and 1:100) were analyzed. When added to the 1:1 mixture, APC was equally inefficient in prolonging the clotting time as when added to the proband's plasma. Half of the normal prolongation was observed when testing the 1:10 mixture, whereas the 1:100 mixture behaved like control plasma. Thus the APC-APTT assay did not discriminate between the presence of 50 % and 100 % APC resistant factor molecules suggesting the method to be a useful screening method for the identification of carrier states.

Since the measured lack of substantial prolongation of the clotting time is not related to Factors VIIIₐ and Vₐ or a Protein C inhibitor of the immunoglobulin type or a defective Protein S - APC interaction the effect is likely to be associated with a hitherto unrecognized coagulation factor.

The APC-APTT assay according to the invention has also been run on plasma samples from about 100 patients with diagnosed thrombosis. About 10% of the patients gave shortening of their clotting times compared to the standard. No apparent heredity could be seen. None of the patients had been found positive in other assays for the determination of-coagulation disorders.

An APC-APTT method similar to method 1 under the heading inventive Methods 1 given above has been run on plasma samples from Protein S deficient patients. In this specific mode the APC amount was adjusted so that pooled normal plasmas resulted in a prolongation of the coagulation time of 40 seconds. The new type of patients we have detected then gave a prolongation of coagulation time of 0-15 seconds while plasma from patients with diagnosed Protein S deficiency gave a prolongation time that was close to normal. In order to check further the influence of Protein S deficiency we also assayed normal plasma that had been made deficient in Protein S by immune adsorption. The prolongation time decreased with about 50% which indicates that the prolongation measured for our new patient group is not caused by Protein S deficiency. We have also added Protein S to plasma from the new patient group and run the inventive method on such plasma. The result has been that the prolongation of the coagulatiuon time is not normalised which further supports that the inventive method does not measure Protein S.

By varying the plasma content of the assay medium it was experimentally verified that one should avoid too low plasma concentrations in the final assay medium.

### Restriction digestion of DNA, PCR and hybridization for assaying a Factor VIII gene X-linked inheritance of the disorder.

DNA from three relatives (the proband, his mother and one of his brothers) that were suspected of carrying a gene for the disorder was subjected to PCR with amplification of the Factor VIII gene and subsequent cleavage with Bcl 1 as described previously (Kogan et al., N. Engl. J. Med. 317, 985-90 (1987)).

In the human population this treatment leads to two different fragments (142 kb and 91 kb, respectively). The DNA of an individual will carry genes giving either both fragments or only one fragments. The mother's DNA gave both 142 kb and 91 kb fragments, while one of her sons gave only the 142 kb fragment and the other only the 91 kb fragment. This is a clear indication that the two sons have received different Factor VIII genes from their mother. The disorder traced could thus not be linked to a gene on an X-chromosome.

## Claims

1. An in vitro method for diagnosing in a human thromboembolic diseases caused by, or for determining the risk of a human to acquire manifestation of, a blood coagulation disorder designated APC resistance and recognized by an abnormally low anticoagulant response to exogenous activated Protein C (abbreviated APC) even in presence of normal levels of functional Protein S, Factors Va and VIIIa, which are normally degraded by APC, and absence of lupus anticoagulants, said method comprising determining for a plasma sample comprising coagulation factors and derived from said human, the anticoagulant activity of exogenous APC by measuring the substrate conversion rate obtained for a coagulation enzyme, the activity of which is influenced by APC, by the following steps:
(i) incubating said plasma sample with:
(1) exogenous APC, or exogenous Protein C together with current exogenous reagents to transform the exogenous Protein C to APC, wherein the concentrations used in the final assay medium being 25 ng/mL-10 µg/mL for human APC, 10 ng/mL-50 µg/mL for non-human APC, and 5 ng/mL-5 µg/mL for bovine APC used in combination with 100 ng/mL-20 µg/mL bovine protein S;
(2) an exogenous Reagent (I), which at least partially activates the blood coagulation system of said sample and is selected in a manner known per se to cause activation of a coagulation factor used for the measurement in step (ii);
(3) components that are necessary for efficient reaction of the activated coagulation factors introduced by step (i)(2), *i*.*e*. phosholipid(s) and Ca⁺⁺ salt giving a Ca²⁺ concentration of 0.5-30mmol/L in the final assay medium; and if desired,
(4) an exogenous substrate for an enzyme, the activity of said enzyme being influenced by APC;
(ii) directly measuring said substrate conversion rate obtained in (i), and
(iii) comparing the conversion rate measured in step (ii) with a standard value obtained from samples from normal individuals, which samples have been subjected to steps (i) and (ii) under the same conditions as the plasma sample from said human,
in which method a substrate conversion rate obtained for a plasma sample in step (ii), that is higher than the standard value indicates that said human suffers from or runs the risk of acquiring manifestation of said disorder.

2. The method of claim 1, wherein the final assay medium has a patient plasma sample content of at least 10% (v/v).

3. The method of claim 1, wherein the final assay medium has a patient plasma content of at least 20% (v/v).

4. The method of claim 1, wherein the final assay medium has a patient plasma content of at least 35% (v/v).

5. The method of claims 1, 2, 3 or 4, wherein Reagent (I) is an activated partial thromboplastine time (APTT) reagent comprising phospholipid(s) and a contact activator of the intrinsic pathway.

6. The method of any of claims 1 to 5, wherein the plasma sample is incubated with the components of (i) (1), (i) (3) and if desired (i) (4) after a preincubation of the sample with Reagent (I) for a time sufficient to activate the blood coagulation system of the sample.

7. The method of any of claims 1 to 6, wherein the exogenous APC of (i) (1) is comprised of purified human APC.

8. The method of any of claims 1 to 7, wherein the exogenous APC of (i) (1) is comprised of bovine APC.

9. The method of claim 8, wherein the bovine APC is added to the sample together with bovine Protein S.

10. The method of any of claims 1 to 6, wherein the plasma sample is incubated in step (i) with exogenous human Protein C or non-human, e. g. bovine, Protein C.

11. The method of claim 10, wherein Protein S derived from the same species as Protein C is used as further additive in step (i).

12. The method of any of claims 1 to 11, wherein the plasma sample is incubated with the components of (1) to (3) in step (i) whereafter the conversion of endogenous fibrinogen to fibrin is measured in step (ii) via clot formation.

13. The method of claim 1, wherein Reagent (I) is
(a) the components necessary to activate the blood coagulation system of the sample via the intrinsic pathway, such as an APTT reagent, a contact activator, Factor IXₐ, Factor XIₐ, Factor XIIₐ, and/or kallikrein, and / or
(b) the components necessary to activate the blood coagulation system via the extrinsic pathway, e.g. tissue thromboplastin,
and the components according to (i) (1), (i) (3) and (i) (4) are added simultaneously with or, where appropriate, after Reagent (I) has been allowed to incubate with the sample for a sufficient time to activate the intrinsic or the extrinsic pathway.

14. The method according to claim 1, wherein the Reagent (I) is exogenous Factor Xₐ or exogenous Factor IXₐ

15. The method of claim 14, wherein the incubation is performed in the presence of exogenous prothrombin.

16. The method according to claim 1, wherein the Reagent (I) is exogenous Factor X in excess of sample endogenous Factor X and in combination with exogenous Factor IXₐ, possibly together with thrombin.

17. The method according to any of claims 1 and 13 to 16, wherein exogenous substrate is present and its conversion is measured, if necessary together with a fibrin polymerization inhibitor.

18. The method according to claim 17, wherein the exogenous substrate is specific for Factor Xₐ and is present together with a fibrin polymerization inhibitor.

19. The method according to claim 17, wherein the exogenous substrate is specific for thrombin and a fibrin polymerization inhibitor is present.

20. The method according to any of claims 1 and 13 to 19, wherein a fibrin polymerization inhibitor together with the exogenous substrate are present during the incubation and wherein the conversion of the exogenous substrate is measured, said exogenous substrate being a synthetic peptide comprising a detectable group.

21. The method of claim 20, wherein said peptide consists of less than five amino acid residues and a group that is specifically released and becomes analytically detectable upon action of the enzyme, e.g. upon action of thrombin or Factor Xₐ said group being bound in an amide linkage to the carboxy terminal of the peptide and being selected from chromogenic, fluorogenic and chemiluminogenic groups (= chromogenic substrate).

22. The method according to any of claims 1 to 21, wherein at least one of the materials of (i) (1) to (i) (4) is present in lyophilized form that is reconstituted in connection with the assay.

23. The method of claim 1, wherein in step (iii) the conversion rate measured for the plasma sample in step (ii) is also compared with the conversion rate obtained for a plasma sample derived from said human, which sample has been subjected to step (i) and (ii) under the same conditions as the first plasma sample but in absence of the component(s) of (i) (1).

## Patentansprüche

1. In vitro-Verfahren zur Diagnose einer thromboembolischen Erkrankung bei einem Menschen, die verursacht wird durch, oder zur Bestimmung des Risikos eines Menschen, eine Manifestation zu erlangen, einer Blutgerinnungsstörung, die als APC-Resistenz bezeichnet wird, und die erkannt wird durch eine abnormal niedrige Antikoagulationsantwort auf exogenes aktiviertes Protein C (abgekürzt APC) sogar in Gegenwart von normalen Spiegeln an funktionellem Protein S, Faktoren Vₐ und VIIIₐ, die durch APC normal abgebaut werden, und Abwesenheit von Lupus-Antikoagulantien, wobei das Verfahren umfaßt: Bestimmen der Antikoagulationsaktivität von exogenem APC in einer Plasmaprobe, welche Koagulationsfaktoren umfaßt und von einem Menschen stammt, durch Messen der Substratumsetzungsrate, die für ein Koagulationsenzym erhalten wird, dessen Aktivität durch APC beeinflußt wird, durch die folgenden Schritte:
(i) Inkubieren der Plasmaprobe mit:
(1) exogenem APC oder exogenem Protein C zusammen mit gebräuchlichen exogenen Reagenzien zur Umwandlung des exogenen Proteins C in APC, wobei die in dem endgültigen Assaymedium angewandten Konzentrationen 25 ng/ml - 10 µg/ml für menschliches APC, 10 ng/ml - 50 µg/ml für nicht-menschliches APC und 5 ng/ml - 5 µg/ml für Rinder-APC, welches in Kombination mit 100 ng/ml - 20 µg/ml Rinder-Protein S verwendet wird, betragen;
(2) einem exogenen Reagens (I), das das Blutkoagulationssystem der Probe mindestens teilweise aktiviert und in an sich bekannter Weise ausgewählt ist, um die Aktivierung des Koagulationsfaktors, der für die Messung in Schritt (ii) verwendet wird, zu bewirken;
(3) Komponenten, die für die wirksame Umsetzung der in dem Schritt (i)(2) zugeführten aktivierten Koagulationsfaktoren notwendig sind, *d. h.* Phospholipid(e) und Ca⁺⁺-Salz, welches eine Ca²⁺-Konzentration von 0,5 - 30 mMol/l in dem endgültigen Assaymedium ergibt; und gewünschtenfalls
(4) einem exogenen Substrat für ein Enzym, wobei die Aktivität des Enzyms durch APC beeinflußt wird;
(ii) direktes Messen der Substratumsetzungsrate, die in (i) erhalten wird, und
(iii) Vergleichen der Umsetzungsrate, die in Schritt (ii) gemessen wird, mit einem Standardwert, der von Proben von normalen Individuen erhalten wird, wobei die Proben den Schritten (i) und (ii) unter den gleichen Bedingungen, wie die Plasmaprobe von dem Menschen unterworfen worden sind;
wobei in dem Verfahren eine Substratumsetzungsrate, die für eine Plasmaprobe im Schritt (ii) erhalten wird, die höher ist als der Standardwert, anzeigt, daß der Mensch an einer Manifestation der Störung leidet oder die Gefahr läuft, eine Manifestation der Störung zu erlangen.

2. Verfahren nach Anspruch 1, wobei das endgültige Assaymedium einen Patientenplasmaprobengehalt von mindestens 10 % (V/V) besitzt.

3. Verfahren nach Anspruch 1, wobei das endgültige Assaymedium einen Patientenplasmaprobengehalt von mindestens 20 % (V/V) besitzt.

4. Verfahren nach Anspruch 1, wobei das endgültige Assaymedium einen Patientenplasmagehalt von mindestens 35 % (V/V) besitzt.

5. Verfahren nach Anspruch 1, 2, 3 oder 4, wobei das Reagenz (I) ein aktiviertes Partial-Thromboplastin-Zeit-(activated partial thromboplastine time, APPT)-Reagenz ist, das ein Phospholipid/Phospholipide und einen Kontaktaktivator des intrinsischen Weges umfaßt.

6. Verfahren nach einem der Ansprüche 1 bis 5, wobei die Plasmaprobe mit den Komponenten von (i) (1), (i) (3) und, sofern gewünscht, (i) (4) inkubiert wird nach einer Präinkubation der Probe mit Reagenz (I) für eine Zeit, die ausreicht, um das Blutkoagulationssystem der Probe zu aktivieren.

7. Verfahren nach einem der Ansprüche 1 bis 6, wobei das exogene APC von (i) (1) aus gereinigtem menschlichem APC besteht.

8. Verfahren nach einem der Ansprüche 1 bis 7, wobei das exogene APC von (i) (1) aus Rinder-APC besteht.

9. Verfahren nach Anspruch 8, wobei das Rinder-APC der Probe zusammen mit Rinder-Protein S zugesetzt wird.

10. Verfahren nach einem der Ansprüche 1 bis 6, wobei die Plasmaprobe in Schritt (i) mit exogenem menschlichem Protein C oder nicht-menschlichem, z. B. Rinder-, Protein C inkubiert wird.

11. Verfahren nach Anspruch 10, wobei Protein S, das von der gleichen Spezies wie Protein C abstammt, als weiteres Additiv in Schritt (i) verwendet wird.

12. Verfahren nach einem der Ansprüche 1 bis 11, wobei die Plasmaprobe mit den Komponenten von (1) - (3) in Schritt (i) inkubiert wird, wonach die Umsetzung von endogenem Fibrinogen zu Fibrin gemessen wird in Schritt (ii) über die Gerinnselbildung.

13. Verfahren nach Anspruch 1, wobei Reagenz (I) ist:
(a) die Komponenten, die erforderlich sind, um das Blutkoagulationssystem der Probe über den intrinsischen Weg zu aktivieren, wie ein APTT-Reagenz, ein Kontaktaktivator, Faktor IXₐ, Faktor XIₐ, Faktor XIIₐ und/oder Kallikrein, und/oder
(b) die Komponenten, die erforderlich sind, um das Blutkoagulationssystem über den extrinsischen Weg zu aktivieren, z. B. Gewebe-Thromboplastin,
und die Komponenten gemäß (i) (1), (i) (3) und (i) (4) zugesetzt werden gleichzeitig mit oder, wo es angemessen ist, nachdem Reagenz (I) mit der Probe für eine ausreichende Zeit inkubieren gelassen worden ist, um den intrinsischen oder extrinsischen Weg zu aktivieren.

14. Verfahren nach Anspruch 1, wobei das Reagenz (I) der exogene Faktor Xₐ oder der exogene Faktor IXₐ ist.

15. Verfahren nach Anspruch 14, wobei die Inkubation in Gegenwart von exogenem Prothrombin durchgeführt wird.

16. Verfahren nach Anspruch 1, wobei das Reagenz (I) der exogene Faktor X im Überschuß an endogenem Proben-Faktor X und in Kombination mit exogenem Faktor IXₐ, möglicherweise zusammen mit Thrombin, ist.

17. Verfahren nach einem der Ansprüche 1 und 13 bis 16, wobei exogenes Substrat vorhanden ist und seine Umsetzung gemessen wird, wenn erforderlich, zusammen mit einem Fibrin-Polymerisationsinhibitor.

18. Verfahren nach Anspruch 17, wobei das exogene Substrat für Faktor Xₐ spezifisch ist und zusammen mit einem Fibrin-Polymerisationsinhibitor vorhanden ist.

19. Verfahren nach Anspruch 17, wobei das exogene Substrat spezifisch für Thrombin ist und ein Fibrin-Polymerisationsinhibitor vorhanden ist.

20. Verfahren nach einem der Ansprüche 1 und 13 bis 19, wobei ein Fibrin-Polymerisationsinhibitor zusammen mit dem exogenen Substrat während der Inkubation vorhanden ist, und wobei die Umsetzung des exogenen Substrats gemessen wird, wobei das exogene Substrat ein synthetisches Peptid ist, das eine nachweisebare Gruppe umfaßt.

21. Verfahren nach Anspruch 20, wobei das Peptid aus weniger als fünf Aminosäureresten und einer Gruppe besteht, die auf spezifische Weise freigesetzt wird, und auf analytische Weise nach Wirkung des Enzyms nachweisbar wird, wie nach Wirkung von Thrombin oder Faktor Xₐ, wobei die Gruppe in einer Amidbindung an das Carboxylende des Peptids gebunden ist und aus chromogenen, fluorogenen und chemiluminogenen Gruppen (chromogenes Substrat) gewählt ist.

22. Verfahren nach einem der Ansprüche 1 bis 21, wobei mindestens einer der Stoffe von (i) (1) bis (i) (4) in lyophilisierter Form vorhanden ist, die in Verbindung mit dem Assay rekonstruiert wird.

23. Verfahren nach Anspruch 1, wobei in Schritt (iii) die Umsetzungsrate, die für die Plasmaprobe in Schritt (ii) gemessen wird, auch mit der Umsetzungsrate verglichen wird, die für eine Plasmaprobe erhalten wird, die von dem Menschen stammt, wobei die Probe den Schritten (i) und (ii) unter den gleichen Bedingungen wie die erste Plasmaprobe, aber in Abwesenheit der Komponente(n) von (i) (1) unterworfen worden ist.

## Revendications

1. Méthode in vitro pour diagnostiquer chez un humain ou pour déterminer le risque qu'un humain acquière la manifestation d'une anomalie de la coagulation du sang que l'on désigne par résistance à APC et reconnue par une réponse anticoagulante anormalement faible à la Protéine C exogène activée (ayant pour abréviation APC) même en présence de niveaux normaux de la protéine fonctionnelle S, des Facteurs Vₐ et VIIIₐ, qui sont normalement dégradés par APC, et l'absence d'anticoagulants du lupus, ladite méthode comprenant la détermination, pour un échantillon de plasma contenant des facteurs de coagulation et dérivé d'un humain, de l'activité anticoagulante de APC exogène en mesurant le taux de conversion du substrat obtenu pour une enzyme de la coagulation du sang, dont l'activité est influencée par APC, par les étapes suivantes:
(i) incubation dudit échantillon de plasma avec
(1) APC exogène, ou Protéine C exogène avec des réactifs exogènes courants pour transformer la Protéine C exogène en APC, où les concentrations utilisées dans le milieu d'essai final sont de 25 ng/mL-10 µg/mL pour l'APC humaine,
10 ng/mL-50 µg/mL pour l'APC non humaine, et 5 ng/mL-5 µg/mL pour l'APC bovine utilisée en combinaison avec 100 ng/mL-20 µg/mL de protéine S bovine.
(2) un Réactif exogène (I), qui active au moins partiellement le système de la coagulation du sang dudit échantillon et qui est sélectionné d'une manière connue afin de provoquer l'activation d'un facteur de coagulation utilisé pour la mesure à l'étape (ii);
(3) des composant qui sont nécessaires pour une réaction efficace des facteurs activés de coagulation introduits par l'étape (i) (2), tels que un ou des phospholipide(s) et un sel de Ca⁺⁺ donnant une concentration de Ca²⁺ de 0,5-30 mmole/L dans le milieu d'essai final; et, si on le souhaite,
(4) un substrat exogène pour une enzyme, l'activité de ladite enzyme étant influencée par APC;
(ii) mesure directe dudit taux de conversion du substrat obtenu en (i), et
(iii) comparaison du taux de conversion mesuré à l'étape (ii) avec une valeur standard obtenue d'échantillons d'individus normaux, lesquels échantillons ont été soumis aux étapes (i) et (ii) dans les mêmes conditions que l'échantillon de plasma dudit humain;
méthode dans laquelle un taux de conversion du substrat obtenu à partir d'un échantillon de plasma à l'étape (ii) qui est plus élevé que la valeur standard, indique que ledit humain souffre de ou risque d'acquérir la manifestation de ladite anomalie.

2. Méthode de la revendication 1, où le milieu d'essai final a une teneur en échantillon de plasma du patient d'au moins 10% (v/v).

3. Méthode de la revendication 1, où le milieu d'essai final a une teneur en plasma du patient d'au moins 20% (v/v).

4. Méthode de la revendication 1, où le milieu d'essai final a une teneur en plasma du patient d'au moins 35% (v/v).

5. Méthode des revendications 1, 2, 3 ou 4, où le réactif (I) est un réactif du temps de thromboplastine partielle activée (APTT) comprenant un ou des phospholipides et un activateur de contact du trajet intrinsèque.

6. Méthode selon l'une quelconque des revendications 1-5, où l'échantillon de plasma est soumis à incubation avec les composants de (i)(1), (i)(3) et, si on le souhaite, (i)(4) après une préincubation de l'échantillon avec le Réactif (I) pendant un temps suffisant pour activer le système de coagulation du sang de l'échantillon.

7. Méthode selon l'une quelconque des revendications 1-6, où APC exogène de (i)(1) se compose de APC humaine purifiée.

8. Méthode selon l'une quelconque des revendications 1-7, où APC exogène de (i)(1) se compose de APC bovine.

9. Méthode de la revendication 8, où APC bovine est ajoutée à l'échantillon en même temps que la Protéine S bovine.

10. Méthode selon l'une quelconque des revendications 1-6 où l'échantillon de plasma est soumis à incubation à l'étape (i) avec la Protéine C humaine exogène, ou la Protéine C non humaine, eg, bovine.

11. Méthode de la revendication 10, où la Protéine S dérivée de la même espèce que la Protéine C est utilisée comme autre additif à l'étape (i).

12. Méthode selon l'une quelconque des revendications 1-11 où l'échantillon de plasma est soumis à incubation avec les composants de (1)-(3) à l'étape (i), ensuite la conversion du fibrinogène endogène en fibrine est mesurée à l'étape (ii) via formation du caillot.

13. Méthode de la revendication 1, où le Réactif (I) est
(a) les composants nécessaires pour activer-le système de coagulation du sang de l'échantillon via le trajet intrinsèque, comme un réactif de APTT, un activateur de contact, le Facteur IX_{a.}, le Facteur XIₐ, le Facteur XIIₐ et/ou la kallikréine, et/ou
(b) les composants nécessaires pour activer le système de coagulation du sang via le trajet intrinsèque, e.g. la thromboplastine du tissu,
et les composants selon (i)(1), (i)(3) et (i)(4) sont ajoutés simultanément avec ou, lorsque cela est approprié après avoir laissé le Réactif (I) en incubation avec l'échantillon pendant un temps suffisant pour activer le trajet intrinsèque ou extrinsèque.

14. Méthode selon la revendication 1, où le Réactif (I) est le Facteur Xₐ exogène ou le Facteur IXₐ exogène.

15. Méthode de la revendication 14, où l'incubation est accomplie en présence de prothrombine exogène.

16. Méthode selon la revendication 1, où le Réactif (I) est le Facteur X exogène en excès du Facteur X endogène de l'échantillon et en association avec le Facteur IXₐ exogène, éventuellement avec la thrombine.

17. Méthode selon l'une quelconque des revendications 1 et 13-16, où le substrat exogène est présent et sa conversion est mesurée, si nécessaire en même temps qu'un inhibiteur de la polymérisation de la fibrine.

18. Méthode selon la revendication 17 où le substrat exogène est spécifique pour le Facteur Xₐ et est présent en même temps qu'un inhibiteur de la polymérisation de la fibrine.

19. Méthode selon la revendication 17 où le substrat exogène est spécifique de la thrombine et un inhibiteur de la polymérisation de la fibrine est présent.

20. Méthode selon l'une quelconque des revendications 1 et 13-19 où un inhibiteur de la polymérisation de la fibrine en même temps que le substrat exogène sont présents pendant l'incubation et où la conversion du substrat exogène est mesurée, ledit substrat exogène étant un peptide synthétique comprenant un groupe détectable.

21. Méthode de la revendication 20, où ledit peptide se compose de moins de cinq résidus d'acides aminés et d'un groupe qui est spécifiquement libéré et devient analytiquement détectable lors de l'action de l'enzyme, e.g. lors de l'action de la thrombine ou du Facteur Xₐ, ledit groupe étant lié dans une liaison amide au carboxy terminal du peptide et étant sélectionné parmi des groupes chromogènes, fluorogènes et chimioluminogènes (= substrat chromogène).

22. Méthode selon l'une quelconque des revendications 1-21 où au moins l'un des matériels de (i)(1)-(i)(4) est présent sous forme lyophilisée qui est reconstituée pour l'essai.

23. Méthode de la revendication 1, où à l'étape (iii) le taux de conversion mesuré pour l'échantillon de plasma à l'étape (ii) est également comparé au taux de conversion obtenu pour un échantillon de plasma dérivé dudit humain, lequel échantillon a été soumis à l'étape (i) et (ii) dans les mêmes conditions que le premier échantillon de plasma mais en l'absence du ou des composants de (i)(1).
